# EUROPEAN PATENT SPECIFICATION

(11) **EP 3 267 977 B1**
(45) Date of publication and mention of the grant of the patent: **11.08.2021**
(21) Application number: 16762469.1
(22) Date of filing: 10.03.2016
(51) Int. Cl.: A61K 9/10, A61K 9/16, A61K 47/34, A61K 31/366, A61K 31/496

(54) **SOLID DISPERSIONS**
FESTSTOFFDISPERSIONEN
DISPERSIONS SOLIDES

(30) Priority: 12.03.2015 US 201562132277 P
(43) Date of publication of application: 17.01.2018
(73) Proprietor: DuPont Nutrition USA, Inc., Wilmington DE 19805 (US)
(72) Inventor: GUAN, Jian, Shenyang 110002 (CN); MAO, Shirui, Shenyang 110016 (CN); HELGERUD, Trond, N-3400 Lier (NO); ZHANG, Yeli, Princeton, NJ 08540 (US)
(74) Representative: DuPont EMEA
(86) International application number: PCT/US2016/021643
(87) International publication number: WO 2016/145132

(56) References cited:
- EP-A1- 2 601 935
- WO-A1-2004/110431
- WO-A1-2014/032742
- US-A1- 2011 262 534
- G Poovi ET AL: "Development of Domperidone Solid Dispersion Powders Using Sodium Alginate as Carrier", European Journal of Applied Sciences, 1 January 2013 (2013-01-01), pages 36-42, XP055504452, DOI: 10.5829/idosi.ejas.2013.5.2.1113 Retrieved from the Internet: URL:http://idosi.org/ejas/5(2)13/1.pdf
- Haoyu Liu ET AL: "The role of polymers in oral bioavailability enhancement; a review", Polymer, 23 October 2010 (2010-10-23), pages 399-415, XP055474546, DOI: 10.1016/j.polymer.2015.09.026 Retrieved from the Internet: URL:http://globalresearchonline.net/journa lcontents/volume4issue3/Article%20033.pdf

## Description

### FIELD OF THE INVENTION

In one aspect, the present invention is directed to a solid dispersion comprising: (a) a pharmaceutical active ingredient or a nutraceutical active ingredient having a low solubility; and (b) sodium or potassium alginate. In another aspect, the present invention is directed to a drug dosage form prepared from such a solid dispersion.

### BACKGROUND OF THE INVENTION

Many of the new chemical entities which are currently being developed in the pharmaceutical field or nutraceutical field are poorly soluble. As is noted by Tiwari et al, Solid Dispersions: An Overview to Modify Bioavailability of Poorly Water Soluble Drugs; International Journal of PharmTech Research, Vol.1, No. 4, pp. 1338-1349 (2009), oral drug delivery is the simplest and easiest way to deliver drugs, with solid oral dosage forms having several advantages over other types of oral dosage forms. One major problem associated with many drugs which exhibit low solubility is that they are only absorbed in the upper small intestine and therefore possess only a small absorption window. Consequently, if such drugs are not released in this gastrointestinal area, they will exhibit low bioavailability; a concern which makes their release profile critical. Solid dispersions, which can be defined as molecular mixtures of poorly water soluble drugs in hydrophilic carriers, are one strategy that can be employed to enhance the drug release of such poorly soluble drugs.

Among the carriers currently employed to produce solid dispersions are polyvinylpyrrolidone (PVP) and hypromellose acetate succinate (HPMCAS). While these materials can be employed to produce solid dispersions exhibiting acceptable release profiles, in certain instances exposure to heat, light and/or high humidity may adversely affect the release profile observed.

Solid dispersions can be produced by a number of different processes, including the fusion method, the solvent method and the supercritical fluid method (Tiwari et al, cited above). Poovi et al, Development of Domperidone Solid Dispersion Powders Using Sodium Alginate as Carrier, European Journal of Applied Sciences 5 (2): 36-42 (2013) compares the release profiles of 1:1 weight ratios of drug: sodium alginate mixtures of dispersions produced by several of these methods.

While Poovi et al conclude that sodium alginate is a suitable carrier in solid dispersions to improve the solubility and dissolution of a poorly soluble drug like domperidone, such publication provides no motivation to increase the amount of alginate employed in the compositions described therein. Specifically, Poovi et al indicate (in the first paragraph on page 40) that their analysis of the 1:1 drug:alginate dispersions formed "indicates that the drug is uniformly dispersed in the powder formulation". Accordingly, one would assume that there would be no benefit to adding additional alginate to such a formulation. Consequently, it is completely unexpected that the release rate of poorly soluble active ingredients could be materially increased by increasing the amount of alginate employed in such solid dispersions.

### SUMMARY OF THE INVENTION

In one aspect, the present invention is directed to a solid dispersion consisting essentially of:
(a) an active ingredient having a low solubility; and
(b) an alginate selected from the group consisting of sodium alginate and potassium alginate;
wherein the weight ratio of active ingredient: alginate is between 1:1.5 and 1:10;
with the proviso that when said alginate in a sodium alginate having a G content of less than 50% by weight,
if such sodium alginate has a viscosity of less than 200 mPa·s in a 1% aqueous solution at 20° C using a Brookfield type RV with Brookfield RV spindle 2 the weight ratio of active ingredient:alginate is between 1:2.5 and 1:5; and
if such sodium alginate has a viscosity of 200 mPa·s or more in a 1% aqueous solution at 20° C using a Brookfield type RV with Brookfield RV spindle 2 the weight ratio of active ingredient:alginate is between 1:1.5 and 1:3.5.

In yet another aspect, the present invention is directed to a drug dosage form prepared from such a solid dispersion.

### DETAILED DESCRIPTION OF THE INVENTION

In one aspect, the present invention is directed to a solid dispersion consisting essentially of:
(a) an active ingredient having a low solubility; and
(b) an alginate selected from the group consisting of sodium alginate and potassium alginate;
wherein the weight ratio of drug:alginate is between 1:1.5 and 1:10;
with the proviso that when said alginate is a sodium alginate having a G content of less than 50% by weight,
if such sodium alginate has a viscosity of less than 200 mPa·s in a 1% aqueous solution at 20° C using a Brookfield type RV with Brookfield RV spindle 2 the weight ratio of active ingredient:alginate is between 1:2.5 and 1:5; and
if such sodium alginate has a viscosity of 200 mPa·s or more in a 1% aqueous solution at 20° C using a Brookfield type RV with Brookfield RV spindle 2 the weight ratio of active ingredient:alginate is between 1:1.5 and 1:3.5.

As is employed herein, the term "active ingredient having low solubility" means a pharmaceutical active ingredient which is classified as a Class II or Class IV active under the Biopharmaceutical Classification System, or a nutraceutical active ingredient having a similar solubility profile. Pursuant to such Classification System, a substance is considered to have low solubility when the highest dose strength is soluble in 250 mL or more of water over a pH range of 1 to 7.5. In one embodiment, solubility can be determined according to the parameters set forth in the following matrix at 20° C:

| Solubility in Water | Parts of water solvent required for 1 part of solute | Solubility range in water (mg/mL) |
|---|---|---|
| Very Soluble | <1 | ≧ 1000 |
| Freely Soluble | from 1 to 10 | 100-1000 |
| Soluble | from 10 to 30 | 33-100 |
| Sparingly Soluble | from 30 to 100 | 10-33 |
| Slightly Soluble | from 100 to 1000 | 1-10 |
| Very Slight Soluble | from 1000 to 10,000 | 0.1-1 |
| Practically Insoluble | ≧ 10,000 | <0.1 |

For the purposes of the present invention, an active ingredient having low solubility includes any active ingredient that falls into the categories: very slightly soluble, and practically insoluble as set forth in the above matrix, although the formulation method described in this invention could increase the solubility of an active ingredient that falls into the categories sparingly soluble and slightly soluble into one of the more soluble categories described above.

As is employed herein, the terms "pharmaceutical active ingredient" includes veterinary drugs as well as those intended for human use. The term "nutraceutical active ingredient having low solubility" refers to a nutraceutical compound which meets the solubility criteria for a low solubility drug defined above.

The low solubility active ingredient may be at least one selected, for example, from: a nonsteroidal anti-inflammatory drug including acetaminophen, acetylsalicylic acid, ibuprofen, fenbuprofen, fenoprofen, flurbiprofen, indomethacin, naproxen, etodolac, ketoprofen, dexibuprofen, piroxicam or aceclofenac; an immunosuppressant or atopic dermatitis drug including cyclosporin, tacrolimus, rapamycin, mycophenolate or pimecrolimus; a calcium channel blocker including nifedipine, nimodipine, nitrendipine, nilvadipine, felodipine, amlodipine or isradipine; an angiotensin II antagonist including valsartan, eprosartan, irbesartan, candesartan, telmisartan, olmesartan or losartan; a cholesterol synthesis-inhibiting hypolipidemic agent including atorvastatin, lovastatin, simvastatin, fluvastatin, rosuvastatin or pravastatin; a cholesterol metabolism- and secretion-promoting hypolipidemic agent including gemfibrozil, fenofibrate, etofibrate or bezafibrate; an antidiabetic drug including pioglitazone, rosiglitazone or metformin; a lipase inhibitor including orlistat; an antifungal agent including itraconazole, amphotericin B, terbinafine, nystatin, griseofulvin, fluconazole or ketoconazole; a hepatoprotective drug including biphenyl dimethyl dicarboxylate, silymarin or ursodeoxycholic acid; a gastrointestinal drug including sofalcone, omeprazole, pantoprazole, famotidine, itopride or mesalazine; an antiplatelet agent including cilostazol or clopidogrel; an osteoporosis drug including raloxifene; an antiviral drug including acyclovir, famciclovir, lamivudine or oseltamivir; an antibiotic including clarithromycin, ciprofloxacin or cefuroxime; an antiasthmatic or antihistamine drug including pranlukast, budesonide or fexofenadine; a hormone drug including testosterone, prednisolone, estrogen, cortisone, hydrocortisone or dexamethasone; an anticancer drug including paclitaxel, docetaxel, paclitaxel derivatives, doxorubicin, adriamycin, daunomycin, camptothecin, etoposide, teniposide or busulfan; salts thereof; and pharmaceutical derivatives thereof. Specifically, it may be at least one selected from naproxen, tacrolimus, valsartan, simvastatin, fenofibrate, itraconazole, biphenyl dimethyl dicarboxylate, silymarin, sofalcone, pantoprazole, cilostazol, salts thereof and pharmaceutical derivatives thereof.

The alginate employed in the composition of this invention is in the form of a sodium or potassium salt. When potassium alginate is employed the weight ratio of poorly soluble active ingredient: alginate may range between 1:1.5 and 1:10. More typically, such dispersions comprise a weight ratio of poorly soluble active ingredient:alginate of between 1:2 and 1:5.

When a sodium alginate having a guluronate ("G") content of at least 50 weight percent or more (based upon the total weight of guluronate and mannuronate) is employed, the weight ratio of poorly soluble active ingredient: alginate may range between 1:1.5 and 1:10. More typically, such dispersions comprise a weight ratio of poorly soluble active ingredient:alginate of between 1:2 and 1:5.

When a sodium alginate having a G content of less than 50 weight percent is employed, the weight ratio of poorly soluble active ingredient:alginate will vary depending upon the viscosity of the particular sodium alginate employed. Specifically, if such sodium alginate has a viscosity of less than 200 mPa·s in a 1% aqueous solution at 20° C using a Brookfield type RV (e.g., RVT, RVF, RVTDV) with Brookfield RV spindle 2, the weight ratio of active ingredient:alginate may range between 1:2.5 and 1:5. If such sodium alginate has a viscosity of 200 mPa·s or more in a 1% aqueous solution at 20° C using a Brookfield type RV (e.g., RVT, RVF, RVTDV) with Brookfield RV spindle 2, the weight ratio of active ingredient:alginate may range between 1:1.5 and 1:3.5.

The solid dispersions of this invention may be produced by any method conventionally employed to produce solid dispersions. These include the solvent evaporation method, the melting or fusion method, the solvent melt method (all of which are described in Poovi et al, discussed above); as well as the supercritical fluid method (as described in Tiwari et al, discussed above). The use of the solvent evaporation method is typically employed.

The present disclosure further provides a drug dosage form produced from the solid dispersion of the present invention.

Such drug dosage form may be a granule, powder, syrup, liquid, suspension, tablet, capsule, troche or pill for oral administration, or transdermal agent, lotion, ophthalmic ointment, ointment, plaster, cataplasm, cream, paste, suspension, liquid, injection or suppository for parenteral administration. Such drug dosage form may additionally comprise excipients which are conventionally employed in pharmaceutical compositions, and may be prepared by methods familiar to one of ordinary skill in the art.

Typical excipients which may be employed in the drug dosage form of this invention include fillers and lubricants. These excipients are employed in conventional amounts which are well known to one of ordinary skill in the art.

Suitable fillers include calcium carbonate (Barcroft, Cal-Carb, CalciPure, Destab, MagGran, Millicarb, Pharma-Carb, Precarb, Sturcal, Vivapres Ca), calcium phosphate, dibasic anhydrous (A-TAB, Di-Cafos A-N, Emcompress Anhydrous, Fujicalin), calcium phosphate, dibasic dihydrate (Cafos, Calipharm, Calstar, Di-Cafos, Emcompress), calcium phosphate tribasic (Tri-Cafos, TRI-CAL WG, TRI-TAB), calcium sulphate (Destab, Drierite, Snow White, Cal-Tab, Compactrol, USG Terra Alba), cellulose powdered (Arbocel, Elcema, Sanacel, Solka-Floc), silicified microcrystalline cellulose (ProSolv), cellulose acetate, compressible sugar (Di-Pac), confectioner's sugar, dextranes (Candex, Emdex), dextrin (Avedex, Caloreen, Crystal Gum, Primogran W), dextrose (Caridex, Dextrofin, Lycadex PF, Roferose, Tab fine D-100), fructose (Advantose, Fructamyl, Fructofin, Krystar), kaolinLion, Sim 90), lactitol (Finlac ACX, Finlac DC, Finlac MCX)5 lactose (Aero Flo 20, Aero Flo 65, Anhydrox, CapsuLac, Fast-Flo, FlowLac, GranuLac, InhaLac, Lactochem, Lactohale, Lactopress, Microfine, Microtose, Pharmatose, Prisma Lac, Respitose, SacheLac, SorboLac, Super-Tab, Tablettose, Wyndale, Zeparox), magnesium carbonate, magnesium oxide (MagGran MO), maltodextrin (C*Dry MD, Glucidex, Glucodry, Lycatab DSH, Maldex, Maltagran, Maltrin, Maltrin QD, Paselli MD 10 PH, Star-Dri), maltose (Advantose 100), mannitol (Mannogem, Pearlitol), microcrystalline cellulose (Avicel PH, Celex, Celphere, Ceolus KG, Emcocel, Ethispheres, Fibrocel, Pharmacel, Tabulose, Vivapur), polydextrose (Litesse), simethicone (Dow Corning Q7-2243 LVA, Cow Corning Q7-2587, Sentry Simethicone), sodium alginate (Kelcosol, Keltone, Protanal), sodium chloride (Alberger), sorbitol (Liponec 70-NC, Liponic 76-NC, Meritol, Neosorb, Sorbifin, Sorbitol Instant, Sorbogem), starch (Aytex P, Fluftex W, Instant Pure-Cote, Melojel, Meritena Paygel 55, Perfectamyl D6PH, Pure-Bind, Pure-Cote, Pure-Dent, Pure-Gel, Pure-Set, Purity 21, Purity 826, Tablet White), pregelatinized starch (Instastarch, Lycatab C, Lycatab PGS, Merigel, National 78-1551, Pharma-Gel, Prejel, Sepistab ST 200, Spress B820, Starch 1500 G, Tablitz, Unipure LD, Unipure WG220), sucrose, trehalose and xylitol (Klinit, Xylifm, Xylitab, Xylisorb, Xylitolo).

The term 'filler' is sometimes used interchangeably with the term 'diluent'. However, the term 'filler' is generally used for solid formulations whereas the term 'diluent' is used in liquid formulations.

Suitable lubricants include calcium stearate (HyQual), glycerine monostearate (Capmul GMS-50, Cutina GMS, Imwitor 191 and 900, Kessco GMS5 Lipo GMS 410, 450 and 600, Myvaplex 600P, Myvatex, Protachem GMS-450, Rita GMS, Stepan GMS, Tegin, Tegin 503 and 515, Tegin 4100, Tegin M, Unimate GMS), glyceryl behenate (Compritol 888 ATO), glyceryl palmitostearate Precirol ATO 5), hydrogenated castor oil (Castorwax, Castorwax MP 70, Castorwax MP 80, Croduret, Cutina HR, Fancol, Simulsol 1293), hydrogenated vegetable oil type I (Akofine, Lubritab, Sterotex, Dynasan P60, Softisan 154, Hydrocote, Lipovol HS-K, Sterotex HM), magnesium lauryl sulphate, magnesium stearate, medium-chain triglycerides (Captex 300, Captex 355, Crodamol GTC/C, Labrafac CC, Miglyol 810, Miglyol 812, Myritol, Neobee M5, Nesatol, Waglinol 3/9280), poloxamer (Lutrol, Monolan, Pluronic, Supronicm Synperonic), polyethylene glycol (Carbowax, Carbowax Sentry, Lipo, Lipoxol, Lutrol E, Pluriol E), sodium benzoate (Antimol), sodium chloride (Alberger), sodium lauryl sulphate (Elfan 240, Texapon K1 2P), sodium stearyl fumarate (Pruv, Alubra), stearic acid (Crodacid E570, Emersol, Hystrene, Industrene, Kortacid 1895, Pristerene), talc (Altaic, Luzenac, Luzenac Pharma, Magsil Osmanthus, Magsil Star, Superiore), sucrose stearate (Surfhope SE Pharma D-1803 F) and zinc stearate (HyQual).

In some preferred embodiments, the drug dosage form of this invention comprises an enteric coating. Enteric coatings which may be employed include, but are not limited to, those based upon shellac, starch and amylose acetate phthalates, styrene-maleic acid copolymers, cellulose acetate succinate, cellulose acetate phthalate (CAP), polyvinylacetate phthalate (PVAP), hydroxypropylmethylcellulose phthalate (grades HP-50 and HP-55), ethylcellulose, fats, butyl stearate, and methacrylic acid-methacrylic acid ester copolymers with acid ionizable groups (including "ACRYLEZE®" and "EUDRAGIT®").

In such enteric embodiments it is useful to note the percent release at 30 minutes (as a criteria for fast onset) and at 90 minutes (for complete release characterization). Enteric (or delayed release) efficacy is typically measured in accordance with monograph section 701, Disintegration, of The United States Pharmaceutical Convention.

It is to be understood that each component, compound, substituent, or parameter disclosed herein is to be interpreted as being disclosed for use alone or in combination with one or more of each and every other component, compound, substituent, or parameter disclosed herein.

It is also to be understood that each amount/value or range of amounts/values for each component, compound, substituent, or parameter disclosed herein is to be interpreted as also being disclosed in combination with each amount/value or range of amounts/values disclosed for any other component(s), compounds(s), substituent(s), or parameter(s) disclosed herein and that any combination of amounts/values or ranges of amounts/values for two or more component(s), compounds(s), substituent(s), or parameters disclosed herein are thus also disclosed in combination with each other for the purposes of this description.

It is further understood that each lower limit of each range disclosed herein is to be interpreted as disclosed in combination with each upper limit of each range disclosed herein for the same component, compounds, substituent, or parameter. Thus, a disclosure of two ranges is to be interpreted as a disclosure of four ranges derived by combining each lower limit of each range with each upper limit of each range. A disclosure of three ranges is to be interpreted as a disclosure of nine ranges derived by combining each lower limit of each range with each upper limit of each range, etc. Furthermore, specific amounts/values of a component, compound, substituent, or parameter disclosed in the description or an example is to be interpreted as a disclosure of either a lower or an upper limit of a range and thus can be combined with any other lower or upper limit of a range or specific amount/value for the same component, compound, substituent, or parameter disclosed elsewhere in the application to form a range for that component, compound, substituent, or parameter.

### EXAMPLES

The following Examples are provided to illustrate the invention in accordance with the principles of this invention, but are not to be construed as limiting the invention in any way except as indicated in the appended claims.

### Example 1

### Preparation of Lovastatin: High G Sodium Alginate Solid Dispersions

Solid dispersions of lovastatin in alginate (Protanal LFR5/60, a sodium alginate having a G content of 65-75% by weight, and a viscosity of 3.5-7 mPa·s in a 1% solution; and a viscosity of 300-700 mPa·s in a 10% solution) were prepared using a solvent evaporation technique at the weight ratios (lovastatin:alginate) indicated in Table 1 below as follows. The required amount of drug was weighed and dissolved in 10 mL ethanol at 60° C followed by dropping into carriers with kneading to prepare paste. The paste was then dried under 40° C over a period of 12 hours to remove the solvents. The resultant solid dispersions were collected and pulverized using a mortar and pestle, and stored in a desiccator at room temperature.

### In Vitro Dissolution Studies

*In vitro* dissolution studies of the lovastatin:alginate solid dispersions produced above were carried out using the paddle method equipped with a USP II apparatus at 37.5 ±0.5° C and a paddle speed of 50 rpm. The appropriate amount of lovastatin solid dispersion (containing 60 mg lovastatin) was added into 900mL dissolution medium (0.05M pH6.8 phosphate buffer with 0.1% SDS as non-sink condition). Samples (6 mL) were collected at predetermined time intervals and immediately filtered through a 0.15 µm syringe filter, the first 2 mL of filtrate was discarded and the remaining filtrate was collected for analysis at 237nm using a UV spectrophotometer (UV-2000, UNIC Instrument Corp., Shanghai, China). Meanwhile, an equal volume of fresh dissolution medium was added. All experiments were carried out in triplicate, and the average results of such studies are summarized in Table 1 below.

**Table 1**

| Lovastatin:LFR 5/60 Dispersion | | | | | |
|---|---|---|---|---|---|
| Time (minutes) | Cumulative Release (%) | | | | |
| | Drug: Alginate Ratio | | | | |
| | 1:1 | 1:2 | 1:3 | 1:4 | 1:5 |
| 0 | 0 | 0 | 0 | 0 | 0 |
| 5 | 65.8 | 69.5 | 71.5 | 78.8 | 69.1 |
| 10 | 79.3 | 85.9 | 82.6 | 91.5 | 82.2 |
| 15 | 85.9 | 91.1 | 90.5 | 95.8 | 92.9 |
| 30 | 88.2 | 91.4 | 93.3 | 101.3 | 96.8 |
| 60 | 89.1 | 91.9 | 98.9 | 101.5 | 96.6 |
| 90 | 90.3 | 91.9 | 99.0 | 100.9 | 97.2 |

The above results demonstrate that increasing the amounts of high G sodium alginate in the dispersions above a 1:1 ratio results in enhanced release of the drug in the solid dispersion.

### Example 2

### Lovastatin:Potassium Alginate Solid Dispersions

Employing the method described in Example 1, solid dispersions of lovastatin in potassium alginate (KF200FTS, a potassium alginate having a G content of 60-70% by weight, and a viscosity of 200-400 mPa·s in a 1% solution) were prepared using the solvent evaporation technique described in Example 1 at the weight ratios (lovastatin:alginate) indicated in Table 2 below.

The lovastatin:potassium alginate solid dispersions prepared were tested in *in vitro* dissolution studies as described in Example 1. The results of such testing are summarized in Table 2.

**Table 2**

| Lovastatin:KF200FTS Dispersion | | | | | |
|---|---|---|---|---|---|
| Time (minutes) | Cumulative Release (%) | | | | |
| | Drug: Alginate Ratio | | | | |
| | 1:1 | 1:2 | 1:3 | 1:4 | 1:5 |
| 0 | 0 | 0 | 0 | 0 | 0 |
| 5 | 24.5 | 21.6 | 23.1 | 29.2 | 21.7 |
| 10 | 32.5 | 30.0 | 37.8 | 45.0 | 31.7 |
| 15 | 40.0 | 38.9 | 50.1 | 60.4 | 45.0 |
| 30 | 57.0 | 57.4 | 67.3 | 72.0 | 58.4 |
| 60 | 67.2 | 77.7 | 81.2 | 87.6 | 76.5 |
| 90 | 72.6 | 89.3 | 87.3 | 92.3 | 80.9 |

The above results demonstrate that increasing the amounts of potassium alginate in the dispersions above a 1:1 ratio results in enhanced release of the drug in the solid dispersion.

### Example 3

### Lovastatin:Low G/Low Viscosity Sodium Alginate Solid Dispersions

Employing the method described in Example 1, solid dispersions of lovastatin in a low G/low viscosity sodium alginate (Protanal CR8133, a sodium alginate having a G content of 37-40% by weight, and a viscosity of 15-45 mPa·s in a 1% solution) were prepared using the solvent evaporation technique described in Example 1 at the weight ratios (lovastatin:alginate) indicated in Table 3 below.

The lovastatin: low G/low viscosity sodium alginate solid dispersions prepared were tested in *in vitro* dissolution studies as described in Example 1. The results of such testing are summarized in Table 3.

**Table 3**

| Lovastatin:Protanal CR8133 Dispersion | | | | | |
|---|---|---|---|---|---|
| Time (minutes) | Cumulative Release (%) | | | | |
| | Drug: Alginate Ratio | | | | |
| | 1:1 | 1:2 | 1:3 | 1:4 | 1:5 |
| 0 | 0 | 0 | 0 | 0 | 0 |
| 5 | 46.6 | 52.7 | 53.5 | 54.1 | 42.8 |
| 10 | 66.6 | 67.9 | 68.2 | 69.1 | 59.2 |
| 15 | 76.1 | 74.8 | 80.2 | 84.9 | 70.3 |
| 30 | 88.7 | 85.3 | 87.6 | 93.6 | 84.8 |
| 60 | 94.3 | 87.9 | 92.3 | 97.8 | 94.4 |
| 90 | 95.1 | 89.3 | 95.6 | 102.5 | 95.5 |

The above results demonstrate that increasing the amounts of a low G/low viscosity sodium alginate in the dispersions above a 1:1 ratio to drug:alginate ratios in the range of from 1:2.5 to 1:5 results in enhanced release of the drug in the solid dispersion.

### Example 4

### Lovastatin:Low G/High Viscosity Sodium Alginate Solid Dispersions

Employing the method described in Example 1, solid dispersions of lovastatin in a low G/high viscosity sodium alginate (Protanal CR8223, a sodium alginate having a G content of 37-40% by weight, and a viscosity of 300-450 mPa·s in a 1% solution) were prepared using the solvent evaporation technique described in Example 1 at the weight ratios (lovastatin:alginate) indicated in Table 4 below.

The lovastatin: low G/high viscosity sodium alginate solid dispersions prepared were tested in *in vitro* dissolution studies as described in Example 1. The results of such testing are summarized in Table 4.

**Table 4**

| Lovastatin:Protanal CR8223 Dispersion | | | | | |
|---|---|---|---|---|---|
| Time (minutes) | Cumulative Release (%) | | | | |
| | Drug:Alginate Ratio | | | | |
| | 1:1 | 1:2 | 1:3 | 1:4 | 1:5 |
| 0 | 0 | 0 | 0 | 0 | 0 |
| 5 | 36.1 | 40.8 | 56.2 | 41.4 | 31.2 |
| 10 | 50.3 | 56.1 | 70.9 | 58.9 | 42.3 |
| 15 | 55.3 | 68.5 | 77.3 | 63.7 | 55.1 |
| 30 | 66.4 | 76.4 | 84.0 | 73.7 | 67.3 |
| 60 | 81.9 | 87.7 | 94.0 | 79.0 | 75.1 |
| 90 | 93.5 | 94.3 | 97.6 | 89.6 | 75.7 |

The above results demonstrate that increasing the amounts of a low G/high viscosity sodium alginate in the dispersions above a 1:1 ratio to drug:alginate ratios in the range of from 1:1.5 to 1:3.5 results in enhanced release of the drug in the solid dispersion.

### Example 5

### Preparation of Itraconazole:Alginate Solid Dispersions

Solid dispersions of itraconazole in alginate LFR5/60 were prepared using the solvent evaporation technique described in Example 1 at weight ratios (itraconazole: carrier) of 1:3, 1:4 and 1:5. The required amount of drug was weighed and dissolved in 6mL dichloromethane followed by dropping into carriers with kneading to prepare a paste. The paste was then dried under 40 ° C over a period of 12 hours to remove the solvents. The resultant solid dispersions were collected and pulverized using a mortar and pestle, and stored in a desiccator at room temperature.

### In Vitro Dissolution Studies

*In vitro* dissolution studies of the itraconazole:alginate solid dispersions produced above were carried out using the paddle method equipped with a USP II apparatus at 37.5 ±0.5° C and a paddle speed of 50 rpm. Appropriate amount of solid dispersion (containing itraconazole 50 mg) was added into 900 mL dissolution medium (0.05M pH6.8 phosphate buffer with 0.6% SDS as non-sink condition). The concentration of itraconazole was determined at 261 nm. The average results of three replicates are listed in Table 5.

**Table 5**

| Itraconazole:LFR 5/60 Dispersion | | | |
|---|---|---|---|
| Time (minutes) | Cumulative Release (%) | | |
| | Drug:Alginate Ratio | | |
| | 1:3 | 1:4 | 1:5 |
| 0 | 0 | 0 | 0 |
| 5 | 16.32 | 40.24 | 33.96 |
| 10 | 30.94 | 46.32 | 44.84 |
| 30 | 38.02 | 55.28 | 55.28 |

### Example 6

### Comparison With Polyvinylpyrrolidone ("PVP") Dispersions and Coarse Drug

### Preparation of PVP Dispersion and Coarse Drug Mixture

A solid dispersion of lovastatin in PVP (Kollidon 29/32; BASF) was prepared by the solvent evaporation method using a RE-52AA rotary evaporator (YARONG Co. Ltd, Shanghai, China). The required amount of lovastatin and PVP to produce a 1:4 mixture were weighed and dissolved in alcohol in a round bottom flask and then removed the solvent using a rotary evaporator under 45°C. The sample was further dried for 2h in a vacuum desiccator and milled in a mortar. The powder was collected and stored in a desiccator at room temperature

The physical mixture was prepared by mixing lovastatin and alginate LFR5/60 at the ratio of 1:4.

### In Vitro Dissolution Studies

An *in vitro* dissolution study of the PVP sample was undertaken following the procedure described in Example 1. The results of such study along with the results for the 1:4 lovastatin: alginate solid dispersions prepared in Example 1 and of the drug alone are summarized in Table 6 below:

**Table 6**

| 1:4 Lovastatin:Dispersant Testing | | | | |
|---|---|---|---|---|
| Time (min) | Cumulative Release (%) | | | |
| | Dispersant Tested | | | |
| | Drug Alone | PVP | LFR 5/60 | CR 8133 |
| 0 | 0 | 0 | 0 | 0 |
| 5 | 13.52 | 48.30 | 78.80 | 54.00 |
| 10 | 18.29 | 64.58 | 91.50 | 69.10 |
| 15 | 22.81 | 72.77 | 95.80 | 84.90 |
| 30 | 29.77 | 81.44 | 101.30 | 93.60 |

### Example 7

### Comparative Testing of Alginate Solid Dispersion With Hypromellose Acetate Succinate (HPMCAS) and Polyvinyl Pyrolidone (PVP) Dispersions under Stress Conditions

### A) Preparation of lovastatin/alginate, lovastatin/PVPS630, and lovastatin/HPMCAS solid dispersions:

Solid dispersions of lovastatin (Hubei Xinyinhe Pharmaceutical) in alginate (Protanal LFR 5/60; FMC Corporation), in hypromellose acetate succinate (HPMCAS-MF; Shin-Etsu Chemical Co. Ltd) and polyvinyl pyrolidone (PVPS630; ISP Technologies, Inc.) were prepared by a solvent evaporation method using a RE-52AA rotary evaporator (YARONG Co. Ltd, Shanghai, China) in drug/carriers weight ratios ranging from 1:1 to 1:5., The required amount of lovastatin and carriers were weighed and dissolved in ethanol in a round bottom flask and then the solvent was removed using a rotary evaporator at 60°C. The samples were further dried for 12 hours in a vacuum desiccator and milled in a mortar. The lovastatin dispersion powder was collected and stored in a desiccator at room temperature.

### B) Determination of Optimum Drug Carrier Ratios

The optimum release drug:carrier release rate for the lovastatin/alginate, lovastatin/PVPS630, and lovastatin/HPMCAS solid dispersions produced in step A was determined by an in vitro dissolution study carried out using the paddle method equipped with a USP II apparatus at 37.5±0.5°C and a paddle speed of 50 rpm. An amount of such dispersion containing 60 mg lovastatin was added into 900mL dissolution medium (0.05M pH6.8 phosphate buffer with 0.1%SDS as non-sink condition, 1X). Samples (6 mL) were collected at predetermined time intervals and immediately filtered through a 0.15 µm syringe filter, the first 2 mL of filtrate was discarded and the remaining filtrate was collected for analysis at 237nm using a UV spectrophotometer (Unic2000, shanghai, China). Meanwhile, an equal volume of fresh dissolution medium was added. All experiments were carried out in triplicate.

The results of such testing indicated that the optimum drug:carrier weight ratios were:

| | |
|---|---|
| Lovastatin:Alginate (LFR 5/60) | 1:4 |
| Lovastatin:HPMCAS | 1:4 |
| Lovastatin:PVP | 1:3 |

### C) Tablet Formation:

Tablets containing the solid dispersions at the optimum ratios determined in Step B were formed employing the following compositions:

| Component | Quantity(mg) |
|---|---|
| Lovastatin Dispersion | 100 |
| Ac-Di-Sol (FMC Corporation) | 20 |
| Crospovidone | 20 |
| Magnesium Stearate | 0.8 |
| Microcrystalline Cellulose | 259.2 |

After being subjected to the storage conditions specified in Steps D, E or F below, in vitro dissolution studies of the tablets produced in Step C were carried out using the paddle method equipped with a USP II apparatus at 37.5±0.5°C and a paddle speed of 50 rpm. The solid dispersion based tablets were put into 900mL dissolution medium (0.05M pH6.8 phosphate buffer with 0.1% SDS, sink condition). Samples (6 mL) were collected at predetermined time intervals and then filtered through a 0.45 µm syringe filter. The first 2 mL of filtrate was discarded and the remaining filtrate was collected for analysis at 237nm using a UV spectrophotometer (Unic2000, Shanghai, China). Meanwhile, an equal volume of fresh dissolution medium was added. All experiments were carried out in triplicate.

### D. High Temperature Stability

Tablets containing the optimum drug: carrier ratio determined in Step C were stored at 60° C for five days and for ten days before being subjected to the dissolution test described in Step C. The results of such testing are presented in Tables 7A, 7B and 7C below:

**Table 7A**

| Heat Stability Testing of Lovastatin:LFR 5/60 Alginate (1:4 weight ratio) | | | | | | |
|---|---|---|---|---|---|---|
| Storage at 60° C | Accumulated Release Percent (%) | | | | | |
| | 5 Minutes | 10 Minutes | 15 Minutes | 30 Minutes | 60 Minutes | 90 Minutes |
| 0 day | 54.56 | 76.55 | 77.40 | 78.67 | 82.05 | 86.86 |
| 5 day | 34.28 | 63.12 | 70.42 | 73.60 | 77.81 | 77.82 |
| 10 day | 35.22 | 58.21 | 66.93 | 68.63 | 72.86 | 77.24 |

**Table 7B**

| Heat Stability Testing of Lovastatin:HPMCAS (1:4 weight ratio) | | | | | | |
|---|---|---|---|---|---|---|
| Storage at 60° C | Accumulated Release Percent (%) | | | | | |
| | 5 Minutes | 10 Minutes | 15 Minutes | 30 Minutes | 60 Minutes | 90 Minutes |
| 0 day | 77.53 | 95.0 | 99.99 | 99.11 | 98.68 | 99.99 |
| 5 day | 19.84 | 27.47 | 42.73 | 51.89 | 54.18 | 58.76 |
| 10 day | 20.60 | 28.99 | 30.52 | 34.34 | 38.15 | 44.26 |

**Table 7C**

| Heat Stability Testing of Lovastatin:PVP (1:3 weight ratio) | | | | | | |
|---|---|---|---|---|---|---|
| | Accumulated Release Percent (%) | | | | | |
| | 5 Minutes | 10 Minutes | 15 Minutes | 30 Minutes | 60 Minutes | 90 Minutes |
| 0 day | 35.03 | 42.66 | 53.20 | 67.68 | 79.54 | 89.02 |
| 5 day | 25.94 | 30.52 | 38.92 | 56.47 | 67.91 | 67.91 |
| 10 day | 22.89 | 35.10 | 36.63 | 44.26 | 51.13 | 54.18 |

The data above indicates that the alginate solid dispersion provided better heat stability than did dispersions formed using HPMCAS or PVP as a carrier.

### E. High Humidity Stability

Tablets containing the optimum drug: carrier ratio determined in Step C were stored at 92.5% relative humidity (RH) for five days and for ten days before being subjected to the dissolution test described in Step C. The results of such testing are presented in Tables 8A, 8B and 8C below:

**Table 8A**

| High Humidity Testing of Lovastatin:LFR 5/60 Alginate (1:4 weight ratio) | | | | | | |
|---|---|---|---|---|---|---|
| Days Stored at 92.5% RH | Accumulated Release Percent (%) | | | | | |
| | 5 Minutes | 10 Minutes | 15 Minutes | 30 Minutes | 60 Minutes | 90 Minutes |
| 0 day | 54.56 | 76.55 | 77.40 | 78.67 | 82.05 | 86.86 |
| 5 day | 34.26 | 63.02 | 70.21 | 73.59 | 78.67 | 78.67 |
| 10 day | 37.22 | 59.21 | 68.94 | 70.63 | 74.86 | 78.24 |

**Table 8B**

| High Humidity Testing of Lovastatin:HPMCAS (1:4 weight ratio) | | | | | | |
|---|---|---|---|---|---|---|
| Days Stored at 92.5% RH | Accumulated Release Percent (%) | | | | | |
| | 5 Minutes | 10 Minutes | 15 Minutes | 30 Minutes | 60 Minutes | 90 Minutes |
| 0 day | 77.53 | 95.0 | 99.99 | 99.11 | 98.68 | 99.99 |
| 5 day | 30.52 | 35.10 | 41.21 | 45.78 | 49.60 | 54.94 |
| 10 day | 20.60 | 37.39 | 40.44 | 42.73 | 42.73 | 42.76 |

**Table 8C**

| High Humidity Testing of Lovastatin:PVP (1:3 weight ratio) | | | | | | |
|---|---|---|---|---|---|---|
| Days Stored at 92.5% RH | Accumulated Release Percent (%) | | | | | |
| | 5 Minutes | 10 Minutes | 15 Minutes | 30 Minutes | 60 Minutes | 90 Minutes |
| 0 day | 35.03 | 42.66 | 53.20 | 67.68 | 79.54 | 89.02 |
| 5 day | 28.23 | 39.68 | 42.73 | 49.6 | 54.94 | 56.45 |
| 10 day | 25.18 | 44.26 | 45.78 | 46.55 | 48.84 | 51.89 |

The data above indicates that the alginate solid dispersion provided better heat stability than did dispersions formed using HPMCAS or PVP as a carrier.

### F. Strong Light Stability

Tablets containing the optimum drug:carrier ratio determined in Step C were stored at 4500± 500 lx for five days and for ten days before being subjected to the dissolution test described in Step C. The results of such testing are presented in Tables 9A, 9B and 9C below:

**Table 9A**

| Strong Light Testing of Lovastatin:LFR 5/60 Alginate (1:4 weight ratio) | | | | | | |
|---|---|---|---|---|---|---|
| Days Stored at ∼4500 lx | Accumulated Release Percent (%) | | | | | |
| | 5 Minutes | 10 Minutes | 15 Minutes | 30 Minutes | 60 Minutes | 90 Minutes |
| 0 day | 54.56 | 76.55 | 77.40 | 78.67 | 82.05 | 86.86 |
| 5 day | 43.99 | 62.90 | 70.32 | 73.86 | 76.86 | 78.78 |
| 10 day | 37.22 | 57.22 | 64.94 | 71.64 | 74.86 | 77.86 |

**Table 9B**

| Strong Light Testing of Lovastatin:HPMCAS (1:4 weight ratio) | | | | | | |
|---|---|---|---|---|---|---|
| Days Stored at ∼4500 lx | Accumulated Release Percent (%) | | | | | |
| | 5 Minutes | 10 Minutes | 15 Minutes | 30 Minutes | 60 Minutes | 90 Minutes |
| 0 day | 77.53 | 95.0 | 99.99 | 99.11 | 98.68 | 99.99 |
| 5 day | 24.37 | 38.15 | 41.21 | 42.73 | 45.02 | 47.31 |
| 10 day | 19.84 | 36.63 | 41.21 | 42.03 | 44.26 | 44.26 |

**Table 9C**

| Strong Light Testing of Lovastatin:PVP (1:3 weight ratio) | | | | | | |
|---|---|---|---|---|---|---|
| Days Stored at ∼4500 lx | Accumulated Release Percent (%) | | | | | |
| | 5 Minutes | 10 Minutes | 15 Minutes | 30 Minutes | 60 Minutes | 90 Minutes |
| 0 day | 35.03 | 42.66 | 53.20 | 67.68 | 79.54 | 89.02 |
| 5 day | 33.58 | 41.97 | 53.42 | 57.99 | 58.77 | 63.34 |
| 10 day | 22.13 | 35.10 | 41.21 | 45.78 | 50.36 | 51.89 |

The data above indicates that the alginate solid dispersion provided better light stability than did dispersions formed using HPMCAS or PVP as a carrier.

## Claims

1. A solid dispersion consisting essentially of:
(a) an active ingredient having a low solubility; and
(b) an alginate selected from the group consisting of sodium alginate and potassium alginate;
wherein the weight ratio of active ingredient:alginate is between 1:1.5 and 1:10;
with the proviso that when said alginate is a sodium alginate having a G content of less than 50% by weight,
if such sodium alginate has a viscosity of less than 200 mPa·s in a 1% aqueous solution at 20° C using a Brookfield type RV with Brookfield RV spindle 2 the weight ratio of active ingredient:alginate is between 1:2.5 and 1:5; and
if such sodium alginate has a viscosity of 200 mPa·s or more in a 1% aqueous solution at 20° C using a Brookfield type RV with Brookfield RV spindle 2 the weight ratio of active ingredient:alginate is between 1:1.5 and 1:3.5.

2. The solid dispersion of claim 1 wherein the alginate comprises a potassium alginate.

3. The solid dispersion of claim 2 wherein the weight ratio of active ingredient: alginate is between 1:2 and 1:5.

4. The solid dispersion of claim 1 wherein the alginate comprises a sodium alginate.

5. The solid dispersion of claim 4 wherein the sodium alginate has a G content of 50 weight percent or more.

6. The solid dispersion of claim 5 wherein the weight ratio of active ingredient: alginate is between 1:2 and 1:5.

7. The solid dispersion of claim 4 wherein the alginate salt comprises a sodium alginate having a viscosity of less than 200 mPa·s in a 1% aqueous solution at 20° C using a Brookfield type RV with Brookfield RV spindle 2.

8. The solid dispersion of claim 4 wherein the alginate salt comprises a sodium alginate having a viscosity of 200 mPa·s or more in a 1% aqueous solution at 20° C using a Brookfield type RV with Brookfield RV spindle 2.

9. The solid dispersion of any of claims 1-8 wherein the active ingredient is a nutraceutical or a pharmaceutical.

10. The solid dispersion of claim 9 wherein the poorly soluble active ingredient is a pharmaceutical selected from the group consisting of a nonsteroidal anti-inflammatory drugs, immunosuppressant or atopic dermatitis drugs, calcium channel blockers, angiotensin II antagonists, cholesterol synthesis-inhibiting hypolipidemic agents, cholesterol metabolism- and secretion-promoting hypolipidemic agents, antidiabetic drugs, lipase inhibitors, antifungal agents, hepatoprotective drugs, gastrointestinal drugs, antiplatelet agents, osteoporosis drugs, antiviral drugs, antibiotics, antiasthmatic or antihistamine drugs, hormone drugs and anticancer drugs.

11. The solid dispersion of claim 10 wherein the poorly active ingredient is selected from the group consisting of acetaminophen, acetylsalicylic acid, ibuprofen, fenbuprofen, fenoprofen, flurbiprofen, indomethacin, naproxen, etodolac, ketoprofen, dexibuprofen, piroxicam, aceclofenac, cyclosporin, tacrolimus, rapamycin, mycophenolate, pimecrolimus, nifedipine, nimodipine, nitrendipine, nilvadipine, felodipine, amlodipine, isradipine, valsartan, eprosartan, irbesartan, candesartan, telmisartan, olmesartan, losartan, atorvastatin, lovastatin, simvastatin, fluvastatin, rosuvastatin, pravastatin, gemfibrozil, fenofibrate, etofibrate, bezafibrate, pioglitazone, rosiglitazone, metformin, orlistat, itraconazole, amphotericin B, terbinafine, nystatin, griseofulvin, fluconazole, ketoconazole, biphenyl dimethyl dicarboxylate, silymarin ursodeoxycholic acid, sofalcone, omeprazole, pantoprazole, famotidine, itopride, mesalazine, cilostazol, clopidogrel, raloxifene, acyclovir, famciclovir, lamivudine, oseltamivir, clarithromycin, ciprofloxacin, cefuroxime, pranlukast, budesonide, fexofenadine, testosterone, prednisolone, estrogen, cortisone, hydrocortisone, dexamethasone, docetaxel, paclitaxel, doxorubicin, adriamycin, daunomycin, camptothecin, etoposide, teniposide and busulfan; and pharmaceutical salts thereof.

12. A drug dosage form produced from the solid dispersion of any of claims 1-11.

13. The drug dosage form of claim 12 wherein such drug dosage form comprises an enteric coating.

14. The drug form of claim 12 wherein such drug dosage form is in the form of a granule, powder, suspension, tablet, capsule, troche or pill for oral administration.

15. The drug form of claim 14 wherein such drug dosage form is in the form of a tablet.

## Patentansprüche

1. Feste Dispersion, im Wesentlichen bestehend aus:
(a) einem Wirkstoff, der eine geringe Löslichkeit aufweist; und
(b) einem Alginat, das ausgewählt ist aus der Gruppe bestehend aus Natriumalginat und Kaliumalginat;
wobei das Gewichtsverhältnis von Wirkstoff:Alginat zwischen 1:1, 5 und 1:10 liegt;
mit der Maßgabe, dass, wenn das Alginat ein Natriumalginat mit einem G-Gehalt von weniger als 50 Gew.-% ist,
wenn ein solches Natriumalginat eine Viskosität von weniger als 200 mPa.s in einer 1%igen wässrigen Lösung bei 20°C unter Verwendung eines Typs Brookfield RV mit Brookfield RV Spindel 2 aufweist, das Gewichtsverhältnis von Wirkstoff:Alginat zwischen 1:2,5 und 1:5 liegt; und
wenn ein solches Natriumalginat eine Viskosität von 200 mPa.s oder mehr in einer 1%igen wässrigen Lösung bei 20°C unter Verwendung eines Typs Brookfield RV mit Brookfield RV Spindel 2 aufweist, das Gewichtsverhältnis von Wirkstoff:Alginat zwischen 1:1,5 und 1:3,5 liegt.

2. Feste Dispersion nach Anspruch 1, wobei das Alginat ein Kaliumalginat umfasst.

3. Feste Dispersion nach Anspruch 2, wobei das Gewichtsverhältnis von Wirkstoff:Alginat zwischen 1:2 und 1:5 liegt.

4. Feste Dispersion nach Anspruch 1, wobei das Alginat ein Natriumalginat umfasst.

5. Feste Dispersion nach Anspruch 4, wobei das Natriumalginat ein G-Gehalt von 50 Gew.-% oder mehr aufweist.

6. Feste Dispersion nach Anspruch 5, wobei das Gewichtsverhältnis von Wirkstoff:Alginat zwischen 1:2 und 1:5 liegt.

7. Feste Dispersion nach Anspruch 4, wobei das Alginatsalz ein Natriumalginat umfasst, das eine Viskosität von weniger als 200 mPa.s in einer 1%igen wässrigen Lösung bei 20°C unter Verwendung eines Typs Brookfield RV mit Brookfield RV Spindel 2 aufweist.

8. Feste Dispersion nach Anspruch 4, wobei das Alginatsalz ein Natriumalginat umfasst, das eine Viskosität von 200 mPa.s oder mehr in einer 1%igen wässrigen Lösung bei 20°C unter Verwendung eines Typs Brookfield RV mit Brookfield RV Spindel 2 aufweist.

9. Feste Dispersion nach einem der Ansprüche 1 bis 8, wobei der Wirkstoff ein Nutrazeutikum oder ein Pharmazeutikum ist.

10. Feste Dispersion nach Anspruch 9, wobei der schwerlösliche Wirkstoff ein Pharmazeutikum ist, das ausgewählt ist aus der Gruppe bestehend aus nichtsteroidalen entzündungshemmenden Arzneimitteln, Immunsuppressiva oder Arzneimitteln gegen atopische Dermatitis, Calciumkanalblockern, Angiotensin-II-Antagonisten, Cholesterinsynthese hemmenden hypolipidämischen Mitteln, Cholesterin-Stoffwechsel. und Sekretion fördernden hypolipidämischen Mitteln, antidiabetischen Arzneimitteln, Lipasehemmern, antimykotischen Mitteln, hepatoprotektiven Arzneimitteln, Magen-Darm-Arzneimitteln, gegen Blutplättchen gerichteten Mitteln, Osteoporose-Arzneimitteln, antiviralen Arzneimitteln, Antibiotika, antiasthmatischen Mitteln oder Antihistaminika, Hormon-Arzneimitteln und Antikrebs-Arzneimitteln.

11. Feste Dispersion nach Anspruch 10, wobei der schwerlösliche Wirkstoff ein Pharmazeutikum ist, das ausgewählt ist aus der Gruppe bestehend aus Acetaminophen, Acetylsalicylsäure, Ibuprofen, Fenbuprofen, Fenoprofen, Flurbiprofen, Indomethacin, Naproxen, Etodolac, Ketoprofen, Dexibuprofen, Piroxicam, Aceclofenac, Cyclosporin, Tacrolimus, Rapamycin, Mycophenolat, Pimecrolimus, Nifedipin, Nimodipin, Nitrendipin, Nilvadipin, Felodipin, Amlodipin, Isradipin, Valsartan, Eprosartan, Irbesartan, Candesartan, Telmisartan, Olmesartan, Losartan, Atorvastatin, Lovastatin, Simvastatin, Fluvastatin, Rosuvastatin, Pravastatin, Gemfibrozil, Fenofibrat, Etofibrat, Bezafibrat, Pioglitazon, Rosiglitazon, Metformin, Orlistat, Itraconazol, Amphotericin B, Terbinafin, Nystatin, Griseofulvin, Fluconazol, Ketoconazol, Biphenyldimethyldicarboxylat, Silymarin Ursodesoxycholsäure, Sofalcon, Omeprazol, Pantoprazol, Famotidin, Itoprid, Mesalazin, Cilostazol, Clopidogrel, Raloxifen, Acyclovir, Famciclovir, Lamivudin, Oseltamivir, Clarithromycin, Ciprofloxacin, Cefuroxim, Pranlukast, Budesonid, Fexofenadin, Testosteron, Prednisolon, Östrogen, Cortison, Hydrocortison, Dexamethason, Docetaxel, Paclitaxel, Doxorubicin, Adriamycin, Daunomycin, Camptothecin, Etoposid, Teniposid und Busulfan; und pharmazeutischen Salzen davon.

12. Arzneimittel-Dosierungsform, die hergestellt ist aus der festen Dispersion nach einem der Ansprüche 1 bis 11.

13. Arzneimittel-Dosierungsform nach Anspruch 12, wobei eine solche Arzneimittel-Dosierungsform eine magensaftresistente Beschichtung umfasst.

14. Arzneimittelform nach Anspruch 12, wobei eine solche Arzneimittel-Dosierungsform in der Form eines Granulats, Pulvers, Suspension, Tablette, Kapsel, Pastille oder Pille zur oralen Verabreichung vorliegt.

15. Arzneimittelform nach Anspruch 14, wobei eine solche Arzneimittel-Dosierungsform in der Form einer Tablette vorliegt.

## Revendications

1. Dispersion solide constituée essentiellement de:
(a) un ingrédient actif ayant une solubilité faible; et
(b) un alginate choisi dans le groupe constitué d'alginate de sodium et d'alginate de potassium;
dans laquelle le rapport en poids de l'ingrédient actif:alginate est entre 1:1,5 et 1:10;
sous réserve que lorsque ledit alginate est un alginate de sodium possédant un contenu G de moins de 50% en poids,
si cet alginate de sodium a une viscosité de moins de 200 mPa.s dans une solution aqueuse à 1% à 20°C en utilisant un appareil Brookfield type RV avec une broche Brookfield RV 2, le rapport en poids de l'ingrédient actif:alginate soit entre 1:2,5 et 1:5; et
si cet alginate de sodium a une viscosité de 200 mPa.s ou plus dans une solution aqueuse à 1% à 20°C en utilisant un appareil Brookfield type RV avec une broche Brookfield RV 2, le rapport en poids de l'ingrédient actif:alginate soit entre 1:1,5 et 1:3,5.

2. Dispersion solide selon la revendication 1, dans laquelle l'alginate comprend un alginate de potassium.

3. Dispersion solide selon la revendication 2, dans laquelle le rapport en poids de l'ingrédient actif:alginate est entre 1:2 et 1:5.

4. Dispersion solide selon la revendication 1, dans laquelle l'alginate comprend un alginate de sodium.

5. Dispersion solide selon la revendication 4, dans laquelle l'alginate de sodium possède un contenu G de 50 pour cent en poids ou plus.

6. Dispersion solide selon la revendication 5, dans laquelle le rapport en poids de l'ingrédient actif:alginate est entre 1:2 et 1:5.

7. Dispersion solide selon la revendication 4, dans laquelle le sel d'alginate comprend un alginate de sodium ayant une viscosité de moins de 200 mPa.s dans une solution aqueuse à 1% à 20°C en utilisant un appareil Brookfield type RV avec une broche Brookfield RV 2.

8. Dispersion solide selon la revendication 4, dans laquelle le sel d'alginate comprend un alginate de sodium ayant une viscosité de 200 mPa.s ou plus dans une solution aqueuse à 1% à 20°C en utilisant un appareil Brookfield type RV avec une broche Brookfield RV 2.

9. Dispersion solide selon l'une quelconque des revendications 1-8, dans laquelle l'ingrédient actif est un produit nutraceutique ou un produit pharmaceutique.

10. Dispersion solide selon la revendication 9, dans laquelle l'ingrédient actif faiblement soluble est un produit pharmaceutique choisis dans le groupe constitué de: médicaments anti-inflammatoires non stéroïdiens, médicaments immunosuppresseurs ou contre la dermatite atopique, agents bloquant les canaux calciques, antagonistes de l'angiotensine II, agents hypolipidémiques inhibant la synthèse du cholestérol, agents hypolipidémiques favorisant le métabolisme et la sécrétion du cholestérol, médicaments antidiabétiques, inhibiteurs de lipase, agents antifongiques, médicaments hépatoprotecteurs, médicaments gastro-intestinaux, agents antiplaquettaires, médicaments contre l'ostéoporose, médicaments antiviraux, antibiotiques, médicaments antiasthmatiques ou antihistaminiques, médicaments hormonaux et médicaments anti-cancer.

11. Dispersion solide selon la revendication 10, dans laquelle l'ingrédient actif faiblement soluble est choisi dans le groupe constitué de: acétaminophène, acide acétylsalicylique, ibuprofène, fenbuprofène, fénoprofène, flurbiprofène, indométhacine, naproxène, étodolac, kétoprofène, dexibuprofène, piroxicam, acéclofénac, cyclosporine, tacrolimus, rapamycine, mycophénolate, pimécrolimus, nifédipine, nimodipine, nitrendipine, nilvadipine, félodipine, amlodipine, isradipine, valsartan, éprosartan, irbésartan, candésartan, telmisartan, olmésartan, losartan, atorvastatine, lovastatine, simvastatine, fluvastatine, rosuvastatine, pravastatine, gemfibrozil, fénofibrate, étofibrate, bézafibrate, pioglitazone, rosiglitazone, metformine, orlistat, itraconazole, amphotéricine B, terbinafine, nystatine, griséofulvine, fluconazole, kétoconazole, dicarboxylate de biphényldiméthyle, silymarine, acide ursodésoxycholique, sofalcone, oméprazole, pantoprazole, famotidine, itopride, mésalazine, cilostazol, clopidogrel, raloxifène, acyclovir, famciclovir, lamivudine, oseltamivir, clarithromycine, ciprofloxacine, céfuroxime, pranlukast, budésonide, fexofénadine, testostérone, prednisolone, œstrogène, cortisone, hydrocortisone, dexaméthasone, docétaxel, paclitaxel, doxorubicine, adriamycine, daunomycine, camptothécine, étoposide, téniposide et busulfan; et des sels pharmaceutiques de ceux-ci.

12. Forme posologique de médicament produite à partir de la dispersion solide selon l'une quelconque des revendications 1-11.

13. Forme posologique de médicament selon la revendication 12, où cette forme posologique de médicament comprend un enrobage entérique.

14. Forme de médicament selon la revendication 12, où cette forme posologique de médicament est sous la forme d'un granulé, d'une poudre, d'une suspension, d'un comprimé, d'une capsule, d'une pastille ou d'une pilule pour une administration orale.

15. Forme de médicament selon la revendication 14, où cette forme posologique de médicament est sous la forme d'un comprimé.
